(19) Europäisches Patentamt
European Patent Office
Office européen des brevets

(11) **EP 4 068 303 A1**

(12) **EUROPEAN PATENT APPLICATION**
published in accordance with Art. 153(4) EPC

(43) Date of publication:
**05.10.2022 Bulletin 2022/40**

(21) Application number: **20893804.3**

(22) Date of filing: **24.11.2020**

(51) International Patent Classification (IPC):
*G16H 50/20* (2018.01)    *A61B 10/00* (2006.01)
*G06F 17/18* (2006.01)    *G10L 15/10* (2006.01)
*G10L 25/66* (2013.01)

(52) Cooperative Patent Classification (CPC):
**A61B 10/00; G06F 17/18; G10L 15/10;
G10L 25/66; G16H 50/20**

(86) International application number:
**PCT/JP2020/043563**

(87) International publication number:
**WO 2021/106825 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.11.2019 JP 2019212031**

(71) Applicant: **Keio University
Tokyo, 108-8345 (JP)**

(72) Inventors:
• **KISHIMOTO Taishiro
Tokyo 160-8582 (JP)**

• **LIANG Kuo-ching
Tokyo 160-8582 (JP)**
• **YOSHIMURA Michitaka
Tokyo 160-8582 (JP)**
• **YOSHIMURA Momoko
Tokyo 160-8582 (JP)**
• **FUJITA Takanori
Tokyo 160-8582 (JP)**
• **MIMURA Masaru
Tokyo 160-8582 (JP)**

(74) Representative: **Vossius & Partner
Patentanwälte Rechtsanwälte mbB
Siebertstraße 3
81675 München (DE)**

(54) **DISEASE PREDICTION DEVICE, PREDICTION MODEL GENERATION DEVICE, AND DISEASE PREDICTION PROGRAM**

(57)    Provided is a device performing machine learning by extracting an acoustic feature value from conversational voice data and predicting a disease level of a subject on the basis of a disease prediction model to be generated by the machine learning, the device including: a matrix calculation unit 23 calculating a spatial delay matrix using a relation value of a plurality of types of acoustic feature values; and a matrix decomposition unit 24 calculating a matrix decomposition value from the spatial delay matrix, in which a relation value reflecting a non-linear and nonstationary relationship of the feature values can be obtained by calculating at least one of a DCCA coefficient and a mutual information amount as the relation value of the plurality of types of acoustic feature values, and the disease level of the subject can be predicted on the basis of the relation value.

Fig. 2

**Description**

Technical Field

The present invention relates to a disease prediction device, a prediction model generation device, and a disease prediction program, in particular, relates to a technology of predicting a possibility that a subject has a specific disease or a severity and a technology of generating a prediction model for prediction.

Background Art

Depression is a mental disorder characterized by a depressive mood, reduced motivation/interest/mental activity/appetite, continuous anxiety/tension/frustration/tiredness, sleeplessness, and the like, and is caused by the overlap of mental stress or physical stress. The recovery is fast as the treatment starts early, and thus, it is important to have an early diagnosis and an early treatment. There are various diagnostic criteria for the depression, and a diagnostic method using machine learning is also proposed (for example, refer to Patent Document 1).

In a system described in Patent Document 1, at least one speech feature is calculated from speech patterns collected from patients, a statistical model for providing a score or evaluation with respect to a depressed state of the patient is learned on the basis of at least a part of the calculated speech feature, and a mental state of the patient is determined by using the statistical model. In Patent Document 1, as an example of the speech feature that is used in the machine learning, a rhythm feature, a low-level feature calculated from a short speech sample (for example, a length of 20 milliseconds), and a high-level temporary feature calculated from a long speech sample (for example, a speech level) are disclosed.

As a specific example of the rhythm feature, a voice break duration, measured values of pitches and energy over various extraction regions, mel frequency cepstral coefficients (MFCCs), novel cepstral features, temporary fluctuation parameters (for example, a speaking rate, a prominence in a duration, the distribution of peaks, the length and the period of a pause, a syllable duration, and the like), speech periodicity, a pitch fluctuation, and a voice/voiceless ratio are disclosed.

In addition, as a specific example of the low-level feature, damped oscillator cepstral coefficients (DOCC), normalized modulation cepstral coefficients (NMCCs), medium duration speech amplitudes (MMeDuSA) feature, gammatone cepstral coefficients (GCCs), a deep TV, and a voice acoustic feature (acoustic phonetic: for example, formant information, an average Hilbert envelope curve, periodic and non-periodic energy in a subband, and the like) are disclosed.

Further, as a specific example of the high-level temporary feature, an inclination feature, a Dev feature, an energy contour (En-con) features, a pitch-related feature, and an intensity-related feature are disclosed.

In a depression evaluation model described in Patent Document 1, three classifiers (a Gaussian backend (GB), decision trees (DT), and a neural network (NN)) are used as an example. In an embodiment using the GB classifier, a specific number of features (for example, four most excellent features) are selected, and a system combination is further executed with respect to the speech of the patient. By using such a depression evaluation model, a more accurate prediction than typical clinical evaluation can be provided.

Patent Document 1: JP-T-2017-532082

Summary of the Invention

Technical Problem

In Patent Document 1 described above, it is described that several speech features are calculated from the speech pattern of the patient, and input to a machine learned depression evaluation model, and thus, the possibility of the depression can be predicted. However, it is only described to use at least one of the calculated speech features. In order to increase the accuracy of the prediction according to the machine learning, the number of feature values to be used is increased as one method, but there is a limit to improve a prediction accuracy only by increasing the number.

In order to further increase the prediction accuracy, for example, it is considered to comprehensively use a plurality of calculated feature values. Even in Patent Document 1 described above, it is described to use a normalized cross-correlation coefficient (refer to Paragraph [0028]). However, the cross-correlation is effective to the analysis of linear correlation between two feature values, but is not capable of grasping a non-linear relationship. In the speaking voice of the patient having the depression, a plurality of feature values have a non-linear relationship, and there is a possibility that the feature values are non-stationarily changed, and thus, it is not possible to sufficiently improve the prediction accuracy only by analyzing the cross-correlation of the feature values.

The invention has been made to solve such problems, and an object thereof is to enable a prediction accuracy of a possibility that a subject has a specific disease or a severity to be improved.

Solution to Problem

In order to attain the object described above, in the invention, a feature value calculation unit calculating a plurality of types of feature values on a time-series basis for each predetermined time unit by analyzing time-series data having a value changing on a time-series basis; a matrix calculation unit calculating a spatial delay matrix including a combination of a plurality of relation values by performing processing of calculating a relation value of the plurality of types of feature values to be included in a moving window having a predetermined time length, with respect to the plurality of types of feature values calculated on a time-series basis for each predetermined time unit, by delaying the moving window by a predetermined delay amount; a matrix operation unit calculating matrix unique data unique to the spatial delay matrix by performing a predetermined operation with respect to the spatial delay matrix; and a disease prediction unit inputting the matrix unique data to a learned disease prediction model and predicting a disease level of a subject are provided, and a relation value relevant to at least one of a detrended cross-correlation analytical value and a mutual information amount is calculated as the relation value of the plurality of types of feature values.

Advantageous Effects of the Invention

According to the invention configured as described above, the relation value including the detrended cross-correlation analytical value or the mutual information amount is calculated on the basis of the plurality of types of feature values calculated for each predetermined time unit from the time-series data having a value changing on a time-series basis, and thus, a relation value reflecting a non-linear and non-stationary relationship in the feature values can be obtained, and a disease level of a subject can be predicted on the basis of the relation value. Accordingly, by using the time-series data of the subject in which the relationship in the plurality of types of feature values is non-linearly and non-stationarily changed over time, the disease level of the subject (a possibility that the subject has a specific disease, a severity, or the like) can be predicted with a higher accuracy.

Brief Description of the Drawings

Fig. 1 is a block diagram illustrating a functional configuration example of a prediction model generation device according to a first embodiment.
Fig. 2 is a block diagram illustrating a functional configuration example of a disease prediction device according to the first embodiment.
Fig. 3 is a diagram describing calculation contents of a spatial delay matrix calculated by a matrix calculation unit of the first embodiment.
Fig. 4 is a diagram describing the calculation contents of the spatial delay matrix calculated by the matrix calculation unit of the first embodiment.
Fig. 5 is a block diagram illustrating a functional configuration example of a prediction model generation device according to a second embodiment.
Fig. 6 is a block diagram illustrating a functional configuration example of a disease prediction device according to the second embodiment.
Fig. 7 is a diagram illustrating an example of a three-dimensional tensor to be generated by a tensor generation unit of the second embodiment.

Mode for Carrying Out the Invention

(First Embodiment)

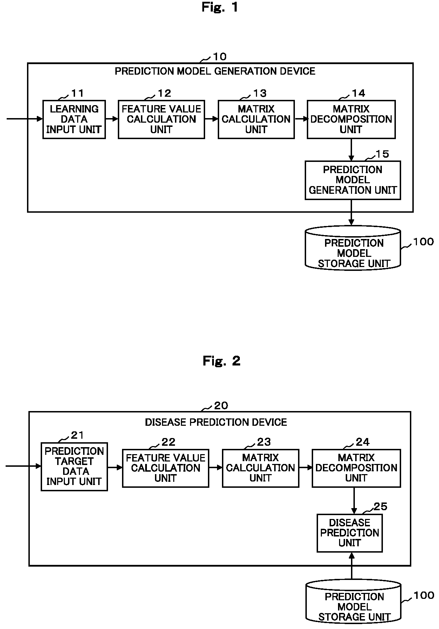

Hereinafter, a first embodiment of the invention will be described on the basis of the drawings. Fig. 1 is a block diagram illustrating a functional configuration example of a prediction model generation device 10 according to the first embodiment. The prediction model generation device 10 according to the first embodiment generates a disease prediction model for predicting a possibility that a subject has a specific disease or a severity in a case where the subject has the specific disease. The disease prediction model is generated by using machine learning. In the first embodiment, as an example, a disease prediction model for predicting a possibility that a subject has depression or a severity is generated.

As illustrated in Fig. 1, the prediction model generation device 10 according to the first embodiment includes a learning data input unit 11, a feature value calculation unit 12, a matrix calculation unit 13, a matrix decomposition unit 14 (corresponding to a matrix operation unit), and a prediction model generation unit 15, as a functional configuration. Such functional blocks 11 to 15 can also be configured by any of hardware, a digital signal processor (DSP), and software.

For example, in a case of being configured by the software, each of the functional blocks 11 to 15 practically includes a CPU, a RAM, a ROM, and the like of a computer, and is attained by operating a disease prediction program stored in a recording medium such as a RAM, a ROM, a hard disk, or a semiconductor memory.

The learning data input unit 11 inputs a series of conversational voice data between a plurality of target people with known disease levels of depression and another person (an example of time-series data having a value changing on a time-series basis) as learning data. Here, the "target people" are patients having the depression and normal people not having the depression, and "another person" having conversation with such target people, for example, is a medical doctor.

The disease level is a value corresponding to the severity of the depression of the target people, and is a value corresponding to a "depression severity evaluation scale" that is generally used as a severity scale for the depression. The depression severity evaluation scale, for example, is a Hamilton depression evaluation scale according to an expert interview (Hamilton depression rating scale: HAM-D), a simple depressive symptom scale to be evaluated by 16 self-completed evaluation scales (quick inventory of depressive symptomatology: QIDS-J), diagnostic criteria of the American Psychiatric Association (the diagnostic and statistical manual of mental disorders: DSM-IV), and the like.

Regarding the patients having the depression, the severity of the depression is specified by the advance diagnosis of the medical doctor or the self-diagnosis, on the basis of the depression severity evaluation scale described above, and the disease level according to the severity is applied to the conversational voice data as a correct answer label. In addition, regarding the normal people not having the depression, the lowest disease level (may be a zero value) is applied to the conversational voice data as a correct answer label. Note that, applying the correct answer label to the conversational voice data does not necessarily indicate that the data of the correct answer label is integrally configured with the conversational voice data, and the conversational voice data and the data of the correct answer label may exist as separate data but may be associated with each other.

The conversational voice data is voice data in which only the speech voice of the target people is extracted from voice data recording a free conversation between the target people and the medical doctor. The free conversation between the target people and the medical doctor, for example, is performed in the form of an interview for approximately 5 to 10 minutes. That is, a conversation in which the medical doctor asks the target people and the target people answer the question is repeated. Then, such a conversation is input by a microphone and recorded, acoustic features of the target people and the medical doctor are extracted from a series of conversational voices by using a known speaker recognition technology, and then, voice data of a speech part of the target people is extracted on the basis of a difference in the acoustic features.

In this case, the voice of the medical doctor may be recorded in advance, and the acoustic feature may be stored, and thus, in a series of conversational voices between the target people and the medical doctor, a voice part having the stored acoustic feature or a feature close thereto may be recognized as the speech voice of the medical doctor, and the other voice part may be extracted as the voice data of the speech voice of the target people. In addition, when recognizing the speaker on the basis of the conversational voice, noise removal processing of extracting only the speaker voice by removing a noise such as an undesired sound or a reverberating sound, and other preprocessings may be performed.

Note that, a method of extracting the voice data of the target people from the conversational voice between the target people and the medical doctor is not limited thereto. For example, in a case where the target people and the medical doctor have a conversation through a call or in a case where a conversation is performed through a remote medical care system or the like in which a terminal and a server are connected through a network, the voice data of the target people can be simply acquired by recording a voice to be input from a telephone or a terminal used by the target people.

The feature value calculation unit 12 calculates a plurality of types of acoustic feature values on a time-series basis for each predetermined time unit by analyzing the conversational voice data (the voice data of the speech voice of the target people) input by the learning data input unit 11. The predetermined time unit indicates individual time unit in which the conversational voice of the target people is divided into short parts, and for example, a time of approximately several dozen milliseconds to several seconds is used as the predetermined time unit. That is, the feature value calculation unit 12 analyzes the conversational voice of the target people by dividing the conversational voice for each predetermined time unit, and calculates the plurality of types of acoustic feature values from each predetermined time unit, and thus, obtains time-series information relevant to the plurality of types of acoustic feature values.

Here, the acoustic feature value to be calculated may be different from the acoustic feature to be extracted when recognizing the speaker as described above. The feature value calculation unit 12, for example, calculates at least two or more of a vocal intensity of the target people, a basic frequency, a cepstral peak prominence (CPP), a formant frequency, and a mel frequency cepstral coefficient (MFCC). In such acoustic feature values, a feature unique to the patient having the depression may be exhibited. Specifically, it is as follows.

- Vocal Intensity: The vocal intensity tends to be low in a case of the depressed patient.
- Basic Frequency: The basic frequency tends to be lower in a case of the patient having the depression, and a repeat count of a minimum period interval for a given length of time tends to be small.
- CPP: CPP is a feature value indicating the properties of breathlessness in the glottis, and is used as a measured value of the severity of a phonation disorder that may occur in the depressed patient.
- Formant Frequency: The formant frequency is a plurality of peaks that are temporally moved in a voice spectrum, indicates the first formant, the second formant, ..., the N-th formant in ascending order of a frequency. It is known that the formant frequency is associated with the shape of the vocal tract, and there is a correlation between the depressive and a sound volume of the formant frequency.
- MFCC: MFCC is a feature value indicating the properties of the vocal tract, and can be an indirect index of the degree of a loss in muscle control of the depressed patients with different severities.

The matrix calculation unit 13 calculates a spatial delay matrix including a combination of a plurality of relation values by performing processing of calculating a relation value of the plurality of types of acoustic feature values to be included in a moving window having a predetermined time length, with respect to the plurality of types of acoustic feature values calculated by the feature value calculation unit 12 on a time-series basis for each predetermined time unit, by delaying the moving window by a predetermined delay amount. Here, the matrix calculation unit 13 calculates at least one of an analytical value of detrended cross-correlation analysis (DCCA) (hereinafter, referred to as a DCCA coefficient) and a mutual information amount, as the relation value of the plurality of types of acoustic feature values. At least one indicates that a spatial delay matrix with the DCCA coefficient as an individual matrix element may be calculated, a spatial delay matrix with the mutual information amount as an individual matrix element may be calculated, or both of the spatial delay matrices may be calculated.

The detrended cross-correlation analysis is one type of fractal analysis, and is a method of removing the trend of the linear relationship to be included in the time-series data with a difference operation, and then, of analyzing the cross-correlation. By performing the analysis by removing the trend of a linear relationship, a non-linear and non-stationary relationship of the plurality of acoustic feature values can be analyzed. That is, it is possible to represent the nonlinear relationship among multiple acoustic features, which is a non-stationary relationship that can vary over time, can be indicated by the time-series information of the DCCA coefficient.

The mutual information amount is an amount indicating the scale of interdependence between two random variables, in a probability theory and an information theory, and can be said as the scale of an information amount shared by two acoustic feature values. For example, the mutual information amount indicates how accurately can the other acoustic feature value be assumed in a case where one acoustic feature value is specified, and for example. In a case where two acoustic feature values are completely independent from each other, the mutual information amount is zero. In other words, the mutual information amount can be said as an index indicating the degree of a linear or non-linear relationship between two acoustic feature values, and the non-linear and non-stationary relationship of the plurality of acoustic feature values can be indicated by the time-series information of the mutual information amount.

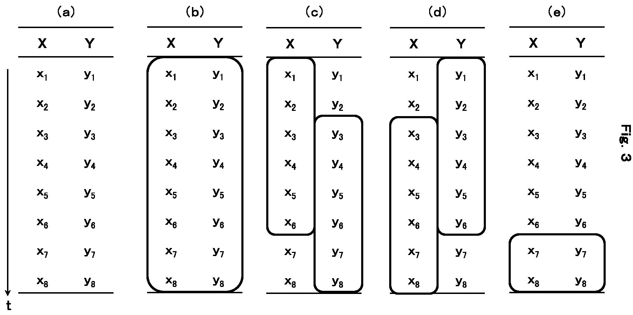

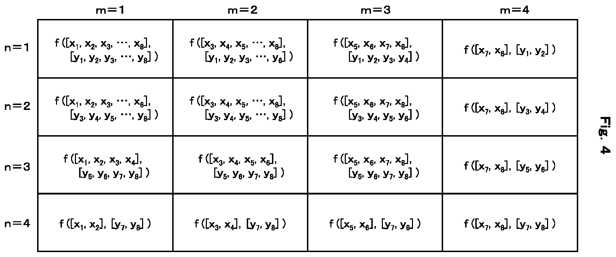

Hereinafter, the calculation contents of the spatial delay matrix calculated by the matrix calculation unit 13 will be described by using Fig. 3 and Fig. 4. Here, in order to simplify the description, an example will be described in which the spatial delay matrix is calculated from two acoustic feature values X and Y.

A first acoustic feature value X calculated by the feature value calculation unit 12 on a time-series basis for each predetermined time unit, and a second acoustic feature value Y calculated on a time-series basis for each predetermined time unit are represented as (Expression 1) and (Expression 2) described below.

$$X = [x_1, x_2, \ldots, x_T] \cdots (\text{Expression 1})$$

$$Y = [y_1, y_2, \ldots, y_T] \cdots (\text{Expression 2})$$

$x_1, x_2, \ldots, x_T$ is time-series information of the first acoustic feature value X calculated for each of T predetermined time units. $y_1, y_2, \ldots, y_T$ is time-series information of the second acoustic feature value Y calculated for each of T predetermined time units.

Fig. 3(a) illustrates that two acoustic feature values X and Y are arranged on a time-series basis in a case of T = 8, and time elapses from top to bottom. T = 8 indicates that the entire interval of the conversational voice of the target people (may be one speech voice in a series of conversations, or may be all speech voices) is divided into 8 parts. For the time-series information of two acoustic feature values X and Y, which are arranged as illustrated in Fig. 3(a), the matrix calculation unit 13 sequentially sets up the moving window having a predetermined time length by delaying a predetermined delay amount. In the example illustrated in Fig. 3, a predetermined delay amount δ is a value with a fixed length, and is set to $\delta = 2$. In addition, a predetermined time length p is a value with a variable length that varies each time when the moving window is set, and is p = 2, 4, 6, 8 (a value that is the integral multiple of $\delta = 2$).

In Fig. 4, a relation value of two acoustic feature values X and Y to be included in a plurality of moving windows to be variable-set is calculated and matrix-represented. In the example of Fig. 4, a square matrix of $4 \times 4$ is calculated as the spatial delay matrix. That is, 16 moving windows are set with respect to the time-series information of Fig. 3(a), and as a result of calculating the relation value of two acoustic feature values X and Y from each of the moving windows, a spatial delay matrix illustrated in Fig. 4 is obtained. As described above, the relation value of two acoustic feature values X and Y is at least one of the DCCA coefficient and the mutual information amount, and an operation for obtaining the relation value is represented by f(X, Y).

In this embodiment, a relation value $A_{mn}$ (m = 1, 2, 3, 4, n = 1, 2, 3, 4) in 16 elements (m, n) of the spatial delay matrix is calculated by an operation represented in (Expression 3) described below.

$$A_{mn} = f(X_m, Y_n) \cdots (\text{Expression 3})$$

$X_m = [X_{1+(m-1)*\delta}, X_{1+(m-1)*\delta+1}, X_{1+(m-1)*\delta+2}, ..., X_{1+(m-1)*\delta+(p-1)}]$
$Y_n = [Y_{1+(n-1)*\delta}, Y_{1+(n-1)*\delta+1}, Y_{1+(n-1)*\delta+2}, ..., Y_{1+(n-1)*\delta+(p-1)}]$
(when m = n = 1, p = 8, when $1 < m, n \le 2$, p = 6, when $2 < m, n \le 3$, p = 4, and when $3 < m, n \le 4$, p = 2)

Fig. 3(b) illustrates a moving window (a thick frame portion) that is set when calculating a relation value $A_{11}$ in the position of an element (1, 1) of the spatial delay matrix illustrated in Fig. 4, on the basis of (Expression 3). That is, in a case of calculating the relation value $A_{11}$ of the element (1, 1), in (Expression 3), the moving window as illustrated in Fig. 3(b) is set as m = 1, n = 1, $\delta = 2$, and p = 8, and the relation value $A_{11} = f(X_1, Y_1)$ is calculated by using the following acoustic feature values $X_1$ and $Y_1$ to be included in the moving window.

$X_1 = [x_1, x_2, x_3, x_4, x_5, x_6, x_7, x_8]$
$Y_1 = [y_1, y_2, y_3, y_4, y_5, y_6, y_7, y_8]$

Fig. 3(c) illustrates a moving window (a thick frame portion) that is set when calculating a relation value $A_{12}$ in the position of an element (1, 2) of the spatial delay matrix illustrated in Fig. 4, on the basis of (Expression 3). That is, in a case of calculating the relation value $A_{12}$ of the element (1, 2), in (Expression 3), the moving window as illustrated in Fig. 3(c) is set as m = 1, n = 2, $\delta = 2$, and p = 6, and the relation value $A_{12} = f(X_1, Y_2)$ is calculated by using the following acoustic feature values $X_1$ and $Y_2$ to be included in the moving window.

$X_1 = [x_1, x_2, x_3, x_4, x_5, x_6]$
$Y_2 = [y_3, y_4, y_5, y_6, y_7, y_8]$

Fig. 3(d) illustrates a moving window (a thick frame portion) that is set when calculating a relation value $A_{21}$ in the position of an element (2, 1) of the spatial delay matrix illustrated in Fig. 4, on the basis of (Expression 3). That is, in a case of calculating the relation value $A_{21}$ of the element (2, 1), in (Expression 3), the moving window as illustrated in Fig. 3(d) is set as m = 2, n = 1, $\delta = 2$, and p = 6, and the relation value $A_{21} = f(X_2, Y_1)$ is calculated by using the following acoustic feature values $X_2$ and $Y_1$ to be included in the moving window.

$X_2 = [x_3, x_4, x_5, x_6, x_7, x_8]$
$X_1 = [y_1, y_2, y_3, y_4, y_5, y_6]$

Fig. 3(e) illustrates a moving window (a thick frame portion) that is set when calculating a relation value $A_{44}$ in the position of an element (4, 4) of the spatial delay matrix illustrated in Fig. 4, on the basis of (Expression 3). That is, in a case of calculating the relation value $A_{44}$ of the element (4, 4), in (Expression 3), the moving window as illustrated in Fig. 3(e) is set as m = 4, n = 4, $\delta = 2$, and p = 2, and the relation value $A_{44} = f(X_4, Y_4)$ is calculated by using the following acoustic feature values $X_4$ and $Y_4$ to be included in the moving window.

$X_4 = [x_7, x_8]$
$Y_4 = [y_7, y_8]$

The matrix decomposition unit 14 calculates the matrix decomposition value as matrix unique data unique to the spatial delay matrix by performing a decomposition operation with respect to the spatial delay matrix calculated by the matrix calculation unit 13. The matrix decomposition unit 14 performs eigenvalue decomposition as an example of the decomposition operation, and calculates an eigenvalue unique to the spatial delay matrix. Note that, as the decomposition operation, other operations such as diagonalization, singular value decomposition, and Jordan decomposition may be performed.

As described above, it can be said that the eigenvalue to be calculated by the feature value calculation unit 12, the matrix calculation unit 13, and the matrix decomposition unit 14 is an intrinsic scalar value reflecting the non-linear and non-stationary relationship with respect to the time-series information of the plurality of types of acoustic feature values to be extracted from the conversational voice of the target people. In this embodiment, the processing of the feature value calculation unit 12, the matrix calculation unit 13, and the matrix decomposition unit 14 is performed with respect to the conversational voice data of each of the plurality of target people that is input by the learning data input unit 11, and thus, the eigenvalues of the plurality of target people are obtained. Then, the eigenvalue is input to the prediction model generation unit 15, and machine learning processing is performed, and thus, the disease prediction model is generated.

The prediction model generation unit 15 generates the disease prediction model for outputting the disease level of the subject when the eigenvalue relevant to the subject is input, by using the eigenvalues of the plurality of target people, which are calculated by the matrix decomposition unit 14, and information of the disease level that is applied to the conversational voice data as the correct answer label. Here, the subject is a person in whom it is unknown whether or not the subject has the depression, and in a case where the subject has the depression, the severity is unknown. The disease prediction model, for example, is a prediction model based on machine learning utilizing a neural network (may be any of a perceptron, a convolutional neural network, a resurgent neural network, a residual network, a RBF network, a probabilistic neural network, a spiking neural network, a complex neural network, and the like).

That is, the prediction model generation unit 15 performs the machine learning by applying a data set of the plurality of target people including the eigenvalues calculated from the conversational voices of the target people and correct answer data of a disease level with respect to the eigenvalue to the neural network as learning data, and thus, adjusts various parameters of the neural network such that when the eigenvalue of a certain target person is input, the disease level as the correct answer corresponding to the eigenvalue is easily output with a high probability. Then, the prediction model generation unit 15 stores the generated disease prediction model in a prediction model storage unit 100.

Note that, here, an example of using the prediction model according to the neural network has been described, but the invention is not limited thereto. For example, the form of the prediction model can also be any one of a regression model (a prediction model based on logistic regression, a support vector machine, or the like), a tree model (a prediction model based on a decision tree, a random forest, a gradient boosting tree, or the like), a Bayesian model (a prediction model based on a Bayesian inference or the like), a clustering model (a prediction model based on a k-neighboring method, hierarchic clustering, non-hierarchic clustering, a topic model, or the like), and the like. The prediction models described here are merely an example, and the invention is not limited thereto.

Fig. 2 is a block diagram illustrating a functional configuration example of the disease prediction device 20 according to the first embodiment. The disease prediction device 20 according to the first embodiment predicts the possibility that the subject has the depression or the severity in a case where the subject has the depression, by using the disease prediction model generated by the prediction model generation device 10 illustrated in Fig. 1.

As illustrated in Fig. 2, the disease prediction device 20 according to the first embodiment includes a prediction target data input unit 21, a feature value calculation unit 22, a matrix calculation unit 23, a matrix decomposition unit 24, and a disease prediction unit 25, as a functional configuration. Each of the functional blocks 21 to 25 can also be configured by any of hardware, DSP, and software. For example, in a case of being configured by the software, each of the functional blocks 21 to 25 practically includes a CPU, a RAM, a ROM, and the like of a computer, and is attained by operating a disease prediction program stored in a recording medium such as a RAM, a ROM, a hard disk, or a semiconductor memory.

The prediction target data input unit 21 inputs a series of conversational voice data between the subject in which the possibility that the subject has the depression or the severity in a case where the subject has the depression is unknown and another person (the medical doctor), as prediction target data. Conversation voice data that is input by the prediction target data input unit 21 is the same as the conversational voice data that is input by the learning data input unit 11, and is the voice data of the speech voice of the subject.

The feature value calculation unit 22, the matrix calculation unit 23, and the matrix decomposition unit 24 execute the same processing as that of the feature value calculation unit 12, the matrix calculation unit 13, and the matrix decomposition unit 14 illustrated in Fig. 1, with respect to the conversational voice data (the voice data of the speech part of the subject) input by the prediction target data input unit 21. Accordingly, a matrix decomposition value (for example, the eigenvalue) reflecting the non-linear and non-stationary relationship with respect to the time-series information the plurality of types of acoustic feature values to be extracted from a conversational voice of a specific subject is calculated.

The disease prediction unit 25 predicts the disease level of the subject by inputting the eigenvalue calculated by the matrix decomposition unit 24 to the learned disease prediction model stored in the prediction model storage unit 100. As described above, the disease prediction model stored in the prediction model storage unit 100 is generated by the prediction model generation device 10 by the machine learning processing using the learning data such that the disease level of the subject is output when the eigenvalue is input.

As described in detail above, in the first embodiment, when the disease level of the subject is predicted on the basis of the disease prediction model to be generated by extracting the acoustic feature value from the conversational voice data and by performing the machine learning, the spatial delay matrix using the relation value of the plurality of types of acoustic feature values is calculated, and the matrix decomposition value is calculated from the spatial delay matrix and used as an input value of the disease prediction model. In particular, in the first embodiment, the relation value relevant to at least one of the DCCA coefficient and the mutual information amount is calculated as the relation value of the plurality of types of acoustic feature values.

According to the first embodiment configured as described above, the relation value including the DCCA coefficient or the mutual information amount is calculated on the basis of the time-series information of the plurality of types of acoustic feature values calculated for each predetermined time unit from the conversational voice data having a value changing on a time-series basis, and thus, the relation value reflecting the non-linear and non-stationary relationship can be obtained, and the disease level of the subject can be predicted on the basis of the relation value. Accordingly, the disease level of the subject (the possibility that the subject has the specific disease, the severity, or the like) can be predicted with a higher accuracy by using the conversational voice data of the subject in which a relationship in the plurality of types of acoustic feature values is non-linearly and non-stationarily changed over time.

Note that, in the first embodiment described above, an example has been described in which the prediction model generation device 10 illustrated in Fig. 1 and the disease prediction device 20 illustrated in Fig. 2 are configured as separate devices, but the invention is not limited thereto. For example, the functional blocks 11 to 14 illustrated in Fig. 1 and the functional blocks 21 to 24 illustrated in Fig. 2 basically perform the same processing, and thus, may be configured as one device having a function of generating the disease prediction model and a function of predicting the disease level by combining the functional blocks. The same applies to a second embodiment described below.

In addition, in the first embodiment described above, a terminal device may include a part of the functional blocks 11 to 15 illustrated in Fig. 1, a server device may include the remaining functional blocks, and the disease prediction model may be generated by cooperation between the terminal device and the server device. Similarly, a terminal device may include a part of the functional blocks 21 to 25 illustrated in Fig. 2, a server device may include the remaining functional blocks, and the disease level may be predicted by cooperation between the terminal device and the server device. The same applies to the second embodiment described below.

In addition, in the first embodiment described above, in order to simplify the description, an example has been described in which one spatial delay matrix is calculated from two acoustic feature values X and Y, and the matrix decomposition value is calculated from the one spatial delay matrix, but two or more spatial delay matrices may be calculated from a combination of three or more acoustic feature values, and the matrix decomposition value may be calculated from each of the two or more spatial delay matrices. For example, in a case of using three acoustic feature values X, Y, and Z, a first spatial delay matrix may be calculated from a combination of the acoustic feature values X and Y, a second spatial delay matrix may be calculated from a combination of the acoustic feature values X and Z, and a third spatial delay matrix may be calculated from a combination of the acoustic feature values Y and Z, and then, the matrix decomposition value may be calculated from each of the three spatial delay matrices. By calculating the eigenvalue on the basis of various combinations of the acoustic feature values, the number of parameters that are used as the input value of the disease prediction model can be increased, and the accuracy of the prediction can be increased.

(Second Embodiment)

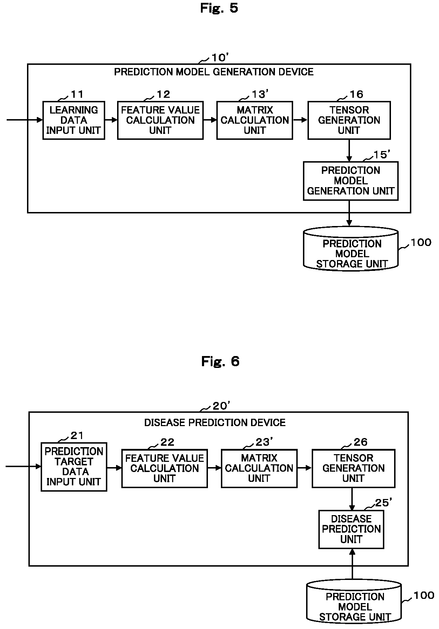

Next, the second embodiment of the invention will be described on the basis of the drawings. Fig. 5 is a block diagram illustrating a functional configuration example of a prediction model generation device 10' according to the second embodiment. The prediction model generation device 10' according to the second embodiment also generates the disease prediction model for predicting the possibility that the subject has the specific disease or the severity in a case where the subject has the specific disease.

In Fig. 5, the constituents with the same reference numerals as those illustrated in Fig. 1 have the same functions, and thus, here, the repeated description will be omitted. As illustrated in Fig. 5, the prediction model generation device 10' according to the second embodiment includes a matrix calculation unit 13', a tensor generation unit 16 (corresponding to the matrix operation unit), and a prediction model generation unit 15', instead of the matrix calculation unit 13, the matrix decomposition unit 14, and the prediction model generation unit 15 illustrated in Fig. 1.

The matrix calculation unit 13' calculates a plurality of spatial delay matrices having the same number of lines and the same number of columns by performing the processing of calculating the relation value (the detrended cross-correlation analytical value or the mutual information amount) of the plurality of types of feature values calculated by the feature value calculation unit 12 on a time-series basis for each predetermined time unit by changing a combination of the feature values.

For example, the matrix calculation unit 13' calculates a spatial delay matrix indicating a relation value between F1 and F2, a spatial delay matrix indicating a relation value between F1 and CPP, a spatial delay matrix indicating a relation value between F1 and I, a spatial delay matrix indicating a relation value between F2 and CPP, a spatial delay matrix indicating a relation value between F2 and I, and a spatial delay matrix indicating a relation value between CPP and I, by using four feature values of a first formant frequency (F1), a second formant frequency (F2), a cepstral peak prominence (CPP), and an intensity (I). Such six spatial delay matrices are the same-dimensional spatial delay matrices having the same number of lines and the same number of columns. Here, an example has been described in which the spatial delay matrix is calculated with respect to all combinations to be obtained by selecting any two from four feature values F1, F2, CPP, and I, but the spatial delay matrix may be calculated with respect to a part of the combinations.

As another example, the matrix calculation unit 13' may calculate the plurality of spatial delay matrices indicating the relation value of MFCCs with respect to all or a part of combinations to be obtained by selecting any two from plurality of mel frequency cepstral coefficients (MFCC). In such a case, the plurality of spatial delay matrices to be generated are the same-dimensional spatial delay matrices having the same number of lines and the same number of columns. The plurality of spatial delay matrices may be calculated with respect to both of all or a part of the combinations to be obtained by selecting any two from four feature values F1, F2, CPP, and I and all or a part of the combinations to be obtained by selecting any two from the plurality of MFCCs.

Further, the matrix calculation unit 13' may calculate one or more difference-series spatial delay matrices by operating a difference in the plurality of spatial delay matrices (hereinafter, referred to as an original spatial delay matrix) calculated as described above. For example, when a plurality of original spatial delay matrices are represented by M1, M2, M3, M4, M5, and M6, one or more difference-series spatial delay matrices are obtained by a difference operation such as M2-M1, M3-M2, M4-M3, M5-M4, and M6-M5.

Here, the matrix calculation unit 13' may calculate a plurality of one-order difference-series spatial delay matrices by operating a difference in the plurality of original spatial delay matrices, and calculate one or more two-order difference-series spatial delay matrices by operating a difference in the plurality of one-order difference-series spatial delay matrices. M2-M1, M3-M2, M4-M3, M5-M4, and M6-M5 exemplified above are the plurality of one-order difference-series spatial delay matrices. The two-order difference-series spatial delay matrix, for example, is obtained by a difference operation such as (M3-M2) - (M2-M1), (M4-M3) - (M3-M2), (M5-M4) - (M4-M3), and (M6-M5) - (M5-M4). Further, a three or higher-order difference-series spatial delay matrix may be calculated.

The tensor generation unit 16 generates a three-dimensional tensor of the relation value (the detrended cross-correlation analytical value or the mutual information amount) of the plurality of types of feature values, as the matrix unique data unique to the spatial delay matrix, by using the plurality of spatial delay matrices calculated by the matrix calculation unit 13'. In a case where the matrix calculation unit 13' calculates the difference-series spatial delay matrix, the tensor generation unit 16 generates the three-dimensional tensor by using the plurality of original spatial delay matrices and one or more difference-series spatial delay matrices calculated by the matrix calculation unit 13'.

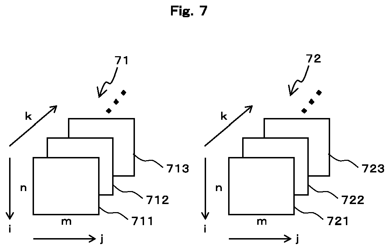

Fig. 7 is a diagram illustrating an example of the three-dimensional tensor (i, j, k) that is generated by the tensor generation unit 16 of the second embodiment. In the example illustrated in Fig. 7, the tensor generation unit 16 generates a first three-dimensional tensor 71 and a second three-dimensional tensor 72. The first three-dimensional tensor 71, for example, is generated by stacking the plurality of spatial delay matrices (the original spatial delay matrix and the difference-series spatial delay matrix) 711, 712, 713, ... to be calculated from four feature values F1, F2, CPP, and I. All of the spatial delay matrices are a matrix having n lines × m columns. The second three-dimensional tensor 72, for example, is generated by stacking the plurality of spatial delay matrices (the original spatial delay matrix and the difference-series spatial delay matrix) 721, 722, 723, ... to be calculated from the plurality of MFCCs. All of the spatial delay matrices are a matrix having n lines × m columns. Note that, the three-dimensional tensor illustrated in Fig. 7 is an example, and the invention is not limited thereto.

The prediction model generation unit 15' generates the disease prediction model for outputting the disease level of the subject when the three-dimensional tensor of the relation value relevant to the subject is input, by using the three-dimensional tensor of the relation value that is generated by the tensor generation unit 16 and the information of the disease level that is applied to the conversational voice data as the correct answer label.

That is, the prediction model generation unit 15' performs the machine learning by applying a data set of the plurality of target people including the three-dimensional tensor of the relation value calculated from the conversational voice of the target people (the patient having the specific disease and the normal people not having the specific disease), and the correct answer data of the disease level with respect to the three-dimensional tensor to the neural network as the learning data, and thus, adjusts various parameters of the neural network such that when a three-dimensional tensor of a certain target person is input, the disease level as a correct answer corresponding to the three-dimensional tensor is easily output with a high probability. Then, the prediction model generation unit 15' stores the generated disease prediction model in the prediction model storage unit 100.

Fig. 6 is a block diagram illustrating a functional configuration example of a disease prediction device 20' according to the second embodiment. The disease prediction device 20' according to the second embodiment predicts the possibility that the subject has the specific disease or the severity in a case where the subject has the specific disease, by using the disease prediction model generated by the prediction model generation device 10' illustrated in Fig. 5. In Fig. 6, the constituents with the same reference numerals as those illustrated in Fig. 2 have the same functions, and thus, here, the repeated description will be omitted.

As illustrated in Fig. 6, the disease prediction device 20' according to the second embodiment includes a matrix calculation unit 23', a tensor generation unit 26, and a disease prediction unit 25', instead of the matrix calculation unit 23, the matrix decomposition unit 24, and the disease prediction unit 25 illustrated in Fig. 2.

The feature value calculation unit 22, the matrix calculation unit 23', and the tensor generation unit 26 execute the same processing as that of the feature value calculation unit 12, the matrix calculation unit 13', and the tensor generation unit 16 illustrated in Fig. 5, with respect to the conversational voice data (the voice data of the speech part of the subject) input by the prediction target data input unit 21. Accordingly, the three-dimensional tensor with the relation value reflecting the non-linear and non-stationary relationship with respect to the time-series information of the plurality of types of acoustic feature values to be extracted from the conversational voice of the specific subject as an element is generated.

The disease prediction unit 25' predicts the disease level of the subject by inputting the three-dimensional tensor of the relation value calculated by the tensor generation unit 26 to the learned disease prediction model stored in the prediction model storage unit 100. As described above, the disease prediction model stored in the prediction model storage unit 100 is generated by the prediction model generation device 10' by the machine learning processing using the learning data such that the disease level of the subject is output when the three-dimensional tensor is input.

As described in detail above, in the second embodiment, the spatial delay matrix with the plurality of relation values reflecting the non-linear and non-stationary relationship of the feature values as an element is input to the disease prediction model in the form of the three-dimensional tensor. That is, unlike the first embodiment in which the eigenvalue that is a scalar value is calculated from the spatial delay matrix and input to the disease prediction model, the spatial delay matrix in which the information amount is not compressed is used as the input of the disease prediction model. Accordingly, a prediction accuracy of the possibility that the subject has the specific disease or the severity can be further improved.

Note that, here, an example of generating the three-dimensional tensor (a case of N = 3 in claims) has been described, but N may be a value 1, 2, or 4 or more. In a case of N = 2, one spatial delay matrix to be generated by the same processing as that in the first embodiment corresponds to a two-dimensional tensor. In a case of N = 1, in one spatial delay matrix, a spatial delay matrix in which the value of any one of m and n is 1 corresponds to a one-dimensional tensor.

In the first and second embodiments described above, an example of obtaining the conversational voice data by recording the free conversation between the target people or the subjects and the medical doctor in the form of an interview has been described, but the invention is not limited thereto. For example, a free conversation of the target people or the subjects in the daily life may be recorded, and the processing described in the embodiments may be performed by using the voice data.

In addition, in the first and second embodiments described above, an example of predicting the disease level of the depression has been described, but the invention is not limited thereto. For example, the disease level may be predicted for individual items relevant to various aspects of the depressed state of the subject, such as sleeping difficulty, a mental symptom of anxiety, a physical symptom of anxiety, psychomotor suppression, and diminished interest.

In addition, in the first and second embodiments described above, the improvement or the degeneration of the depressed state may be grasped by repeatedly performing the prediction of the disease level of the subject periodically or non-periodically.

In addition, in the first and second embodiments described above, an example of calculating at least two or more of the vocal intensity, the basic frequency, CPP, the formant frequency, and MFCC, as the acoustic feature value, has been described, but this is merely an example, and other acoustic feature values may be calculated.

In addition, in the first and second embodiments described above, an example of setting the predetermined delay amount to a fixed length of $\delta$ = 2 has been described, but the invention is not limited thereto. That is, the variation of the eigenvalue to be calculated from the spatial delay matrix may be further increased by calculating the spatial delay matrix with the predetermined delay amount as a variable length.

In addition, in the first and second embodiments described above, an example of predicting the disease level by analyzing the conversational voice data has been described, but data having a value changing on a time-series basis is effective for obtaining the matrix decomposition value by calculating the spatial delay matrix using at least one of the DCCA coefficient and the mutual information amount.

For example, the spatial delay matrix with the relation value including at least one of the DCCA coefficient and the mutual information amount as the individual matrix element can be calculated by analyzing video data obtained by photographing a human face and by extracting a plurality of types of feature values unique to the human face. As the feature value relevant to the face, for example, a ratio, an intensity, and an average duration of an expression (a bland expression, joyfulness, astonishment, angriness, and sadness) in a predetermined time unit, a possibility to move to the next expression, and the like can be used. In addition, as another feature value relevant to the face, things relevant to eye-blink, for example, a blink timing of left and right eyes, a temporal difference, and the like can be used.

In addition, as another example of the data having a value changing on a time-series basis, video data obtained by photographing the motion of a human body (for example, a head, a chest, shoulders, arms, and the like) can also be used. Note that, the time-series data capturing the motion of the human body is not necessarily video data. For example, the time-series data may be time-series data to be detected by an acceleration sensor, an infrared sensor, or the like.

In addition, the calculation of the spatial delay matrix and the calculation of the matrix decomposition value may be performed by using the acoustic feature value extracted from the voice data of the conversational voice, the feature value relevant to the expression or the eye-blink extracted from the video data, and the feature value relevant to the body motion extracted from the video data or the sensor data as a multimodal parameter, and the prediction of the disease level may be performed by using the obtained matrix decomposition value.

In addition, in the first and second embodiments described above, an example of using at least one of the DCCA coefficient and the mutual information amount as the relation value of the acoustic feature values has been described, but it does not intend to use only at least one of the DCCA coefficient and the mutual information amount, and other relation values may be used in combination. For example, a correlation coefficient of cross-correlation effective for grasping a linear relationship in two events can be further calculated, and the spatial delay matrix can also be calculated by adding the correlation coefficient. More specifically, in a case of using the multimodal parameter as described above, the feature value for calculating the relation value by using at least one of the DCCA coefficient and the mutual information amount, and the feature value for calculating the relation value by using the correlation coefficient of the cross-correlation or the other coefficients may be used differently.

In addition, in the first and second embodiments described above, an example of predicting the disease level of the depression as an example of the disease has been described, but the predictable disease is not limited thereto. For example, dementia, insomnia, attention-deficit hyperactivity disorder (ADHD), integration disorder syndrome, a post traumatic stress disorder (PTSD), and other diseases relevant to neuropsychological disturbance can also be predicted.

In addition, both of the first and second embodiments described above are merely a specific example for carrying out the invention, and the technical scope of the invention is not construed to a limited extent by the embodiments. That is, the invention can be carried out in various forms without departing from the gist or the main features thereof.

Reference Signs List

10, 10' Prediction model generation device
11 Learning data input unit
12 Feature value calculation unit
13, 13' Matrix calculation unit
14 Matrix decomposition unit (matrix operation unit)
15, 15' Prediction model generation unit
16 Tensor generation unit (matrix operation unit)
20, 20' Disease prediction device
21 Prediction target data input unit
22 Feature value calculation unit
23, 23' Matrix calculation unit
24 Matrix decomposition unit (matrix operation unit)
25, 25' Disease prediction unit
26 Tensor generation unit (matrix operation unit)
100 Prediction model storage unit

**Claims**

**1.** A disease prediction device **characterized by** comprising:

a feature value calculation unit calculating a plurality of types of feature values on a time-series basis for each predetermined time unit by analyzing time-series data having a value changing on a time-series basis;
a matrix calculation unit calculating a spatial delay matrix including a combination of a plurality of relation values by performing processing of calculating a relation value of the plurality of types of feature values to be included in a moving window having a predetermined time length, the relation value being relevant to at least one of a detrended cross-correlation analytical value and a mutual information amount, with respect to the plurality of types of feature values calculated by the feature value calculation unit on a time-series basis for each predetermined time unit, by delaying the moving window by a predetermined delay amount;

a matrix operation unit calculating matrix unique data unique to the spatial delay matrix by performing a predetermined operation with respect to the spatial delay matrix calculated by the matrix calculation unit; and

a disease prediction unit inputting the matrix unique data calculated by the matrix operation unit to a learned disease prediction model and predicting a disease level of a subject,

wherein the disease prediction model is generated by machine learning processing using learning data such that the disease level of the subject is output when the matrix unique data is input.

**2.** The disease prediction device according to claim 1, **characterized in that**

the matrix operation unit includes a matrix decomposition unit calculating a matrix decomposition value unique to the spatial delay matrix by performing a decomposition operation with respect to the spatial delay matrix calculated by the matrix calculation unit, and

the disease prediction unit inputs the matrix decomposition value calculated by the matrix decomposition unit to the learned disease prediction model and predicts the disease level of the subject.

**3.** The disease prediction device according to claim 1, **characterized in that**

the matrix operation unit includes a tensor generation unit generating an N-dimensional tensor ($N \geq 1$) of the relation value by using one or more spatial delay matrices calculated by the matrix calculation unit, and

the disease prediction unit inputs the N-dimensional tensor generated by the tensor generation unit to the learned disease prediction model and predicts the disease level of the subject.

**4.** The disease prediction device according to claim 3, **characterized in that**

the matrix calculation unit calculates a plurality of spatial delay matrices having the same number of lines and the same number of columns by performing the processing of calculating the relation value by changing a combination of the feature values,

the tensor generation unit generates a three-dimensional tensor of the relation value by using the plurality of spatial delay matrices calculated by the matrix calculation unit, and

the disease prediction unit inputs the three-dimensional tensor generated by the tensor generation unit to the learned disease prediction model and predicts the disease level of the subject.

**5.** The disease prediction device according to claim 4, **characterized in that**

the matrix calculation unit calculates a plurality of original spatial delay matrices having the same number of lines and the same number of columns by performing the processing of calculating the relation value by changing the combination of the feature values, and calculates one or more difference-series spatial delay matrices by operating a difference in the plurality of original spatial delay matrices, and

the tensor generation unit generates the three-dimensional tensor by using the plurality of original spatial delay matrices and the one or more difference-series spatial delay matrices that are calculated by the matrix calculation unit.

**6.** The disease prediction device according to claim 5, **characterized in that**
the matrix calculation unit calculates a plurality of one-order difference-series spatial delay matrices by operating a difference in the plurality of original spatial delay matrices, and calculates one or more two-order difference-series spatial delay matrices by operating a difference in the plurality of one-order difference-series spatial delay matrices.

**7.** The disease prediction device according to any one of claims 1 to 6, **characterized in that**
the feature value calculation unit calculates a plurality of types of acoustic feature values relevant to a speech voice of the subject by analyzing a series of conversational voice data of the subject and another person.

**8.** The disease prediction device according to claim 7, **characterized in that**
the feature value calculation unit calculates at least two or more of vocal intensity of the subject, a basic frequency, a cepstral peak prominence (CPP), a formant frequency, and a mel frequency cepstral coefficient (MFCC).

9. A prediction model generation device **characterized by** comprising:

a learning data input unit inputting time-series data having a value changing on a time-series basis, which is acquired with respect to a plurality of target people with known disease levels, as learning data;

a feature value calculation unit calculating a plurality of types of feature values on a time-series basis for each predetermined time unit by analyzing the time-series data input by the learning data input unit;

a matrix calculation unit calculating a spatial delay matrix including a combination of a plurality of relation values by performing processing of calculating a relation value of the plurality of types of feature values to be included in a moving window having a predetermined time length, the relation value being relevant to at least one of a detrended cross-correlation analytical value and a mutual information amount, with respect to the plurality of types of feature values calculated by the feature value calculation unit on a time-series basis for each predetermined time unit, by delaying the moving window by a predetermined delay amount;

a matrix operation unit calculating matrix unique data unique to the spatial delay matrix by performing a predetermined operation with respect to the spatial delay matrix calculated by the matrix calculation unit; and

a prediction model generation unit generating a disease prediction model for outputting a disease level of a subject when matrix unique data relevant to the subject is input, by using the matrix unique data calculated by the matrix operation unit,

wherein the disease prediction model is generated by performing the processing of the feature value calculation unit, the matrix calculation unit and the matrix operation unit with respect to the time-series data of each of the plurality of target people that is input by the learning data input unit, and by performing machine learning processing by inputting unique data of the plurality of target people to the prediction model generation unit.

10. The prediction model generation device according to claim 9, **characterized in that**

the matrix operation unit includes a matrix decomposition unit calculating a matrix decomposition value unique to the spatial delay matrix by performing a decomposition operation with respect to the spatial delay matrix calculated by the matrix calculation unit, and

the prediction model generation unit generates the disease prediction model for outputting the disease level of the subject when a matrix decomposition value relevant to the subject is input, by using the matrix decomposition value calculated by the matrix decomposition unit.

11. The prediction model generation device according to claim 9, **characterized in that**

the matrix operation unit includes a tensor generation unit generating an N-dimensional tensor (N ≥ 1) of the relation value by using one or more spatial delay matrices calculated by the matrix calculation unit, and

the prediction model generation unit generates the disease prediction model for outputting the disease level of the subject when a three-dimensional tensor relevant to the subject is input, by using the N-dimensional tensor generated by the tensor generation unit.

12. A disease prediction program for allowing a computer to function as:

a feature value calculation means calculating a plurality of types of feature values on a time-series basis for each predetermined time unit by analyzing time-series data having a value changing on a time-series basis;

a matrix calculation means calculating a spatial delay matrix including a combination of a plurality of relation values by performing processing of calculating a relation value of the plurality of types of feature values to be included in a moving window having a predetermined time length, the relation value being relevant to at least one of a detrended cross-correlation analytical value and a mutual information amount, with respect to the plurality of types of feature values calculated by the feature value calculation means on a time-series basis for each predetermined time unit, by delaying the moving window by a predetermined delay amount;

a matrix operation means calculating matrix unique data unique to the spatial delay matrix by performing a predetermined operation with respect to the spatial delay matrix calculated by the matrix calculation means; and

a disease prediction means inputting the matrix unique data calculated by the matrix operation means to a learned disease prediction model that is generated by machine learning processing using learning data such that a disease level of a subject is output when the matrix unique data is input and predicting the disease level of the subject.

**13.** The disease prediction program according to claim 12, **characterized in that**

the matrix operation means includes a matrix decomposition means calculating a matrix decomposition value unique to the spatial delay matrix by performing a decomposition operation with respect to the spatial delay matrix calculated by the matrix calculation means, and
the disease prediction means inputs the matrix decomposition value calculated by the matrix decomposition means to the learned disease prediction model and predicts the disease level of the subject.

**14.** The disease prediction program according to claim 12, **characterized in that**

the matrix operation means includes a tensor generation means generating an N-dimensional tensor ($N \geq 1$) of the relation value by using one or more spatial delay matrices calculated by the matrix calculation means, and
the disease prediction means inputs the N-dimensional tensor generated by the tensor generation means to the learned disease prediction model and predicts the disease level of the subject.

Fig. 1

~10
PREDICTION MODEL GENERATION DEVICE

~11
LEARNING DATA INPUT UNIT

~12
FEATURE VALUE CALCULATION UNIT

~13
MATRIX CALCULATION UNIT

~14
MATRIX DECOMPOSITION UNIT

~15
PREDICTION MODEL GENERATION UNIT

~100
PREDICTION MODEL STORAGE UNIT

Fig. 2

~20
DISEASE PREDICTION DEVICE

~21
PREDICTION TARGET DATA INPUT UNIT

~22
FEATURE VALUE CALCULATION UNIT

~23
MATRIX CALCULATION UNIT

~24
MATRIX DECOMPOSITION UNIT

~25
DISEASE PREDICTION UNIT

~100
PREDICTION MODEL STORAGE UNIT

**Fig. 3**

|  | m=1 | m=2 | m=3 | m=4 |
|---|---|---|---|---|
| n=1 | $f([x_1, x_2, x_3, \cdots, x_8], [y_1, y_2, y_3, \cdots, y_8])$ | $f([x_3, x_4, x_5, \cdots, x_8], [y_1, y_2, y_3, \cdots, y_6])$ | $f([x_5, x_6, x_7, x_8], [y_1, y_2, y_3, y_4])$ | $f([x_7, x_8], [y_1, y_2])$ |
| n=2 | $f([x_1, x_2, x_3, \cdots, x_6], [y_3, y_4, y_5, \cdots, y_8])$ | $f([x_3, x_4, x_5, \cdots, x_8], [y_3, y_4, y_5, \cdots, y_8])$ | $f([x_5, x_6, x_7, x_8], [y_3, y_4, y_5, y_6])$ | $f([x_7, x_8], [y_3, y_4])$ |
| n=3 | $f([x_1, x_2, x_3, x_4], [y_5, y_6, y_7, y_8])$ | $f([x_3, x_4, x_5, x_6], [y_5, y_6, y_7, y_8])$ | $f([x_5, x_6, x_7, x_8], [y_5, y_6, y_7, y_8])$ | $f([x_7, x_8], [y_5, y_6])$ |
| n=4 | $f([x_1, x_2], [y_7, y_8])$ | $f([x_3, x_4], [y_7, y_8])$ | $f([x_5, x_6], [y_7, y_8])$ | $f([x_7, x_8], [y_7, y_8])$ |

Fig. 4

**Fig. 5**

PREDICTION MODEL GENERATION DEVICE — 10'

| 11 LEARNING DATA INPUT UNIT | 12 FEATURE VALUE CALCULATION UNIT | 13' MATRIX CALCULATION UNIT | 16 TENSOR GENERATION UNIT |

15' PREDICTION MODEL GENERATION UNIT

PREDICTION MODEL STORAGE UNIT — 100

**Fig. 6**

DISEASE PREDICTION DEVICE — 20'

| 21 PREDICTION TARGET DATA INPUT UNIT | 22 FEATURE VALUE CALCULATION UNIT | 23' MATRIX CALCULATION UNIT | 26 TENSOR GENERATION UNIT |

25' DISEASE PREDICTION UNIT

PREDICTION MODEL STORAGE UNIT — 100

**Fig. 7**

## INTERNATIONAL SEARCH REPORT

| International application No. |
|---|
| PCT/JP2020/043563 |

**A. CLASSIFICATION OF SUBJECT MATTER**
G16H 50/20(2018.01)i; A61B 10/00(2006.01)i; G06F 17/18(2006.01)i; G10L 15/10(2006.01)i; G10L 25/66(2013.01)i
FI: G16H50/20; G10L15/10 500Z; G10L25/66; A61B10/00 H; G06F17/18 Z
According to International Patent Classification (IPC) or to both national classification and IPC

**B. FIELDS SEARCHED**

Minimum documentation searched (classification system followed by classification symbols)
G16H50/20; A61B10/00; G06F17/18; G10L15/10; G10L25/66

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2002/087434 A1 (COMPUTER KONBINIENSU KK) 07 November 2002 (2002-11-07) page 4, lines 4-12, page 11, lines 10-12, 13, page 30, line 22 to page 31, line 24 | 1-6, 9-14<br>7, 8 |
| Y | JP 2004-240394 A (SENSE IT SMART CORPORATION) 26 August 2004 (2004-08-26) paragraphs [0035]-[0037], [0051]-[0052], fig. 6, 7 | 7, 8 |
| A | JP 2002-306492 A (LECTRONIC NAVIGATION RESEARCH INSTITUTE, AN INDEPENDENT ADMINISTRATIVE INSTITUTION) 22 October 2002 (2002-10-22) entire text, all drawings | 1-14 |

☐ Further documents are listed in the continuation of Box C.  ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 15 February 2021 (15.02.2021) | 22 February 2021 (22.02.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

## INTERNATIONAL SEARCH REPORT
Information on patent family members

| | International application No. |
|---|---|
| | PCT/JP2020/043563 |

| Patent Documents referred in the Report | Publication Date | Patent Family | Publication Date |
|---|---|---|---|
| WO 2002/087434 A1 | 07 Nov. 2002 | US 2004/137639 A1 paragraphs [0022], [0111]-[0114], [0227]-[0230] | |
| JP 2004-240394 A | 26 Aug. 2004 | (Family: none) | |
| JP 2002-306492 A | 22 Oct. 2002 | US 2004/107105 A1 EP 1386584 A1 WO 2002/085215 A1 KR 10-2004-0015092 A IL 158325 A | |

Form PCT/ISA/210 (patent family annex) (January 2015)